(19) Europäisches Patentamt
European Patent Office
Office européen des brevets

(11) **EP 4 349 875 A1**

(12) **EUROPEAN PATENT APPLICATION**
published in accordance with Art. 153(4) EPC

(43) Date of publication:
**10.04.2024 Bulletin 2024/15**

(21) Application number: **22816047.9**

(22) Date of filing: **30.05.2022**

(51) International Patent Classification (IPC):
*C08F 20/06* (2006.01)     *C08F 2/18* (2006.01)
*A61F 13/15* (2006.01)     *A61F 13/53* (2006.01)
*B01J 20/26* (2006.01)

(52) Cooperative Patent Classification (CPC):
**A61F 13/15; A61F 13/53; B01J 20/26; C08F 2/18; C08F 20/06**

(86) International application number:
**PCT/JP2022/021925**

(87) International publication number:
**WO 2022/255300 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2021   JP 2021091473**

(71) Applicant: **Sumitomo Seika Chemicals Co., Ltd.**
**Kako-gun, Hyogo 675-0145 (JP)**

(72) Inventor: **KOGURE, Naoki**
**Himeji-shi, Hyogo 672-8076 (JP)**

(74) Representative: **Mewburn Ellis LLP**
**Aurora Building**
**Counterslip**
**Bristol BS1 6BX (GB)**

(54) **METHOD FOR PRODUCING WATER-ABSORBING RESIN, WATER-ABSORBING RESIN, ABSORBER, AND ABSORBENT ARTICLE**

(57)    Provided is a method for producing a water-absorbing resin having increased water holding capacity, water absorption capacity under load, and DW without pressure. This method for producing a water-absorbing resin produces the same by subjecting a water-soluble ethylenically unsaturated monomer to reverse phase suspension polymerization in a hydrocarbon dispersion medium, the method including: a polymerization step for mixing an aqueous solution containing the water-soluble ethylenically unsaturated monomer, the hydrocarbon dispersion medium, a polymerization initiator, a dispersion stabilizer and an internal crosslinking agent; and a post-crosslinking step in which the polymer obtained in the polymerization step is post-crosslinked. The polymerization initiator includes an azo compound and a peroxide. The water-soluble ethylenically unsaturated monomer contains 70-100 mol% of acrylic acid and salts thereof. The concentration of the water-soluble ethylenically unsaturated monomer in the aqueous solution containing the water-soluble ethylenically unsaturated monomer is 40 mass% or more. The molar ratio of the internal crosslinking agent relative to the azo compound (internal crosslinking agent/azo compound) is 0.05-0.10.

**EP 4 349 875 A1**

**Description**

TECHNICAL FIELD

The present invention relates to a method for producing a water-absorbent resin, a water-absorbent resin, an absorber, and an absorbent article, and more particularly to a method for producing a water-absorbent resin for constituting an absorber to be suitably used for sanitary materials such as disposable diapers, sanitary napkins, and incontinence pads, a water-absorbent resin, an absorber comprising the water-absorbent resin, and an absorbent article.

BACKGROUND ART

In recent years, water-absorbent resins have been widely used in a field of sanitary materials such as disposable diapers, sanitary napkins, and incontinence pads.

As such a water-absorbent resin, a crosslinked product of a polymer of a water-soluble ethylenically unsaturated monomer, more specifically, a crosslinked product of a polymer of a partially neutralized polyacrylic acid is considered to be a preferable water-absorbent resin because it has various advantages, for example, as follows: it has superior water absorption capacity; it can be produced with a constant quality and at a low cost because of the easy industrial availability of acrylic acid, which is its raw material; and it is less prone to suffering from decomposition or degradation (see, for example, Patent Document 1).

An absorbent article such as a disposable diaper, a sanitary napkin, or an incontinence pad is composed of an absorber that absorbs and retains a body fluid such as urine or menstrual blood excreted from the body, the absorber being positioned mainly in a central portion, a liquid-permeable front sheet (top sheet) positioned on the side of the absorbent article that is brought into contact with the body, and a liquid-impermeable rear sheet (back sheet) positioned opposite from the side that is brought into contact with the body. The absorber is usually composed of hydrophilic fibers such as pulp and a water-absorbent resin.

PRIOR ART DOCUMENT

PATENT DOCUMENT

Patent Document 1: Japanese Patent Laid-open Publication No. H3-227301

SUMMARY OF THE INVENTION

PROBLEMS TO BE SOLVED BY THE INVENTION

In such an absorbent article, a high water retention capacity is required for the water-absorbent resin contained in the absorber. By increasing the water retention capacity of the water-absorbent resin, it is possible to improve a phenomenon in which the liquid once absorbed in the absorber reverses (namely, a phenomenon in which the liquid reverses from the absorber; when touching the absorber with a hand, the user feels uncomfortable wetting).

Further, as characteristics of a water-absorbent resin in an absorbent article, a water-absorbent resin having a high water absorption capacity under a load is considered to be preferable because the water-absorbent resin has a high water absorption capacity even in a situation where pressure is applied to the water-absorbent resin inside the absorbent article. In addition, a water-absorbent resin having a high non-pressurization DW is considered to be preferable because the water-absorbent resin can quickly absorb water even in a situation where the absorbent article including the water-absorbent resin is inclined (that is, the absorbent article can quickly absorb flowing liquid).

It, however, has been conventionally known that when a water-absorbent resin is enhanced in water retention capacity, its water absorption capacity under a load and non-pressurization DW decrease. In the use of an absorbent article including a water-absorbent resin having characteristics with a high water retention capacity, in a case where the absorbing surface of the absorbent article is inclined, if the water-absorbent resin has a low non-pressurization DW, a liquid excreted repeatedly is not absorbed sufficiently in spite of the high water retention capacity of the water-absorbent resin, so that a problem that the liquid leaks out of the absorbent article may occur.

Under such circumstances, a main object of the present invention is to provide a method for producing a water-absorbent resin enhanced in water retention capacity, water absorption capacity under a load, and non-pressurization DW.

MEANS FOR SOLVING THE PROBLEM

The present inventors have conducted intensive studies in order to solve the above problems. As a result, the present inventors have found that in a method of producing a water-absorbent resin by reversed phase polymerizing a water-soluble ethylenically unsaturated monomer in a hydrocarbon dispersion medium, a water-absorbent resin having an increased water retention capacity, an increased water absorption capacity under a load, and an increased non-pressurization DW is obtained when the method includes a prescribed polymerization step and a prescribed post-crosslinking step, an azo compound and a peroxide are used in combination as a polymerization initiator, the concentration of the water-soluble ethylenically unsaturated monomer in an aqueous solution containing the water-soluble ethylenically unsaturated monomer is set higher than conventional, and the molar ratio of an internal crosslinking agent to the azo compound is set within a prescribed range. The present invention has been accomplished through further intensive studies based on such findings.

In summary, the present invention provides aspects with the following configurations:

Item 1. A method for producing a water-absorbent resin by performing reversed phase suspension polymerization of a water-soluble ethylenically unsaturated monomer in a hydrocarbon dispersion medium, the method comprising:

a polymerization step of mixing an aqueous solution containing the water-soluble ethylenically unsaturated monomer, the hydrocarbon dispersion medium, a polymerization initiator, a dispersion stabilizer, and an internal crosslinking agent; and
a post-crosslinking step of post-crosslinking the polymer obtained in the polymerization step,
wherein
the polymerization initiator comprises an azo compound and a peroxide,
the water-soluble ethylenically unsaturated monomer has a content of acrylic acid and a salt thereof of 70 mol% or more and 100 mol% or less,
a concentration of the water-soluble ethylenically unsaturated monomer in an aqueous solution containing the water-soluble ethylenically unsaturated monomer is 40% by mass or more, and
a molar ratio of the internal crosslinking agent to the azo compound (the internal crosslinking agent/the azo compound) is 0.05 or more and 0.10 or less.

Item 2. The method for producing a water-absorbent resin according to item 1, wherein a molar ratio of the peroxide to the azo compound (the peroxide/the azo compound) is 0.1 or more and 1.0 or less.
Item 3. The method for producing a water-absorbent resin according to item 1 or 2, wherein in the post-crosslinking step, a molar ratio of the post-crosslinking agent to the water-soluble ethylenically unsaturated monomer (the post-crosslinking agent/the water-soluble ethylenically unsaturated monomer) is 0.00001 or more and 0.001 or less.
Item 4. The method for producing a water-absorbent resin according to any one of items 1 to 3, wherein the water-absorbent resin has the following properties (A) to (C):

(A) a total value of a physiological saline retention capacity and a water absorption capacity under a load of 4.14 kPa is 60 or more;
(B) a total value of the physiological saline retention capacity and a value of non-pressurization DW after 5 minutes is 90 or more;
(C) the physiological saline retention capacity is 44 g/g or more and 70 g/g or less.

Item 5. A water-absorbent resin which is a crosslinked product of a polymer of a water-soluble ethylenically unsaturated monomer, wherein

the water-soluble ethylenically unsaturated monomer has a content of acrylic acid and a salt thereof of 70 mol% or more and 100 mol% or less, and
the water-absorbent resin has the following properties (A) and (B):

(A) a total value of a physiological saline retention capacity and a water absorption capacity under a load of 4.14 kPa is 60 or more;
(B) a total value of the physiological saline retention capacity and a value of non-pressurization DW after 5 minutes is 90 or more;

Item 6. The water-absorbent resin according to item 5, wherein the physiological saline retention capacity is 44 g/g or more and 70 g/g or less.

Item 7. An absorber comprising the water-absorbent resin according to item 5 or 6.
Item 8. An absorbent article comprising the absorber according to item 7.

ADVANTAGES OF THE INVENTION

According to the present invention, it is possible to provide a method for producing a water-absorbent resin having increased water retention capacity, water absorption capacity under load, and non-pressurization DW. Furthermore, the present invention can also provide a water-absorbent resin, an absorber prepared using the water-absorbent resin, and an absorbent article.

BRIEF DESCRIPTION OF THE DRAWINGS

Fig. 1 is a schematic view of a measuring device to be used for measuring the physiological saline absorption capacity of a water-absorbent resin under a load of 4.14 kPa.
Fig. 2 is a schematic view of a measuring device to be used for measuring the non-pressurization DW of a water-absorbent resin.
Fig. 3 is a schematic view for explaining the method of a slope absorption test of an absorbent article.

EMBODIMENTS OF THE INVENTION

In the present description, the term "comprising" includes "consisting essentially of" and "consisting of". In addition, in the present description, the term "(meth) acryl" means "acryl or methacryl", the term "(meth)acrylate" means "acrylate or methacrylate", and the term "(poly)" means both cases with and without the prefix "poly". In addition, in the present description, the term "water-soluble" means that a solubility of 5% by mass or more is exhibited in water at 25°C.
In the present description, two numerical values joined by "to" means a numerical range including the numerical values before and after "to" as a lower limit value and an upper limit value, respectively. When a plurality of lower limit values and a plurality of upper limit values are described separately, an arbitrary lower limit value and an arbitrary upper limit value may be selected and joined by "to".

1. Method for producing water-absorbent resin

The method for producing a water-absorbent resin of the present invention is a method of producing the water-absorbent resin by performing reversed phase suspension polymerization of a water-soluble ethylenically unsaturated monomer in a hydrocarbon dispersion medium. The method for producing a water-absorbent resin of the present invention comprises a polymerization step of mixing an aqueous solution containing a water-soluble ethylenically unsaturated monomer, a hydrocarbon dispersion medium, a polymerization initiator, a dispersion stabilizer, and an internal crosslinking agent, and a post-crosslinking step of post-crosslinking the polymer obtained in the polymerization step. The polymerization initiator comprises an azo compound and a peroxide. The water-soluble ethylenically unsaturated monomer has a content of acrylic acid and a salt thereof of 70 mol% or more and 100 mol% or less. Further, the concentration of the water-soluble ethylenically unsaturated monomer in the aqueous solution containing the water-soluble ethylenically unsaturated monomer is set to 40% by mass or more, and the molar ratio of the internal crosslinking agent to the azo compound (internal crosslinking agent/azo compound) is 0.05 or more and 0.10 or less.
The method for producing a water-absorbent resin of the present invention can suitably produce a water-absorbent resin having an increased water retention capacity, an increased water absorption capacity under a load, and an increased non-pressurization DW owing to possessing those configurations. The water-absorbent resin of the present invention will be hereinafter described in detail.

<Polymerization step>

In the polymerization step, an aqueous solution containing a water-soluble ethylenically unsaturated monomer, a hydrocarbon dispersion medium, a polymerization initiator, a dispersion stabilizer, and an internal crosslinking agent are mixed. As described above, in the method for producing a water-absorbent resin of the present invention, in the polymerization step, the concentration of the water-soluble ethylenically unsaturated monomer in the aqueous solution containing the water-soluble ethylenically unsaturated monomer is set to a high concentration of 40% by mass or more, and the molar ratio of the azo compound of the polymerization initiator and the internal crosslinking agent (internal crosslinking agent/azo compound) is set within a range of 0.05 to 0.10.

As described later, in the present invention, when a polymerization step in which the reversed phase suspension polymerization is performed in two or more multiple stages is employed, it is preferable to, in the polymerization step of at least the first stage, set the concentration of the water-soluble ethylenically unsaturated monomer in the aqueous solution containing the water-soluble ethylenically unsaturated monomer to a high concentration of 40% by mass or more and set the molar ratio of the azo compound of the polymerization initiator and the internal crosslinking agent (internal crosslinking agent/azo compound) within a range of 0.05 to 0.10, and it is more preferable to set, also in the reversed phase suspension polymerization of the second or later stage, to employ these settings.

[Water-soluble ethylenically unsaturated monomer]

Examples of the water-soluble ethylenically unsaturated monomer include (meth)acrylic acid and salts thereof; 2-(meth)acrylamido-2-methylpropanesulfonic acid and salts thereof; nonionic monomers such as (meth)acrylamide, N,N-dimethyl(meth)acrylamide, 2-hydroxyethyl (meth)acrylate, N-methylol(meth)acrylamide, and polyethylene glycol mono(meth)acrylate; and amino group-containing unsaturated monomers such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, from the viewpoint of being readily industrially available. These water-soluble ethylenically unsaturated monomers may be used alone or in combination of two or more.

Among these water-soluble ethylenically unsaturated monomers, acrylic acid and salts thereof are widely used as raw materials of water-absorbent resins. Copolymers of acrylic acid and/or salts thereof with other water-soluble ethylenically unsaturated monomers as mentioned above may also be used. In the present invention, the content of acrylic acid and a salt thereof in the water-soluble ethylenically unsaturated monomer is 70 to 100 mol%. That is, the proportion of acrylic acid and a salt thereof accounting for in all the water-soluble ethylenically unsaturated monomer is 70 to 100 mol%.

The water-soluble ethylenically unsaturated monomer is dispersed in a state of an aqueous solution in a hydrocarbon dispersion medium, and then subjected to reversed phase suspension polymerization. In the present invention, one of the characteristics is that the concentration of the water-soluble ethylenically unsaturated monomer in the aqueous solution containing the water-soluble ethylenically unsaturated monomer is set to such a high concentration. The concentration of the water-soluble ethylenically unsaturated monomer in the aqueous solution is just required to be 40% by mass or more, and is preferably 42% by mass or more, and more preferably 44% by mass or more. The upper limit of the concentration of the water-soluble ethylenically unsaturated monomer is preferably set to a saturation concentration or less, more preferably is 55% by mass or less, still more preferably 50% by mass or less, and further preferably 46% by mass or less.

As the water-soluble ethylenically unsaturated monomer, one in which an acid group (acid group of acrylic acid) is neutralized in advance with an alkaline neutralizing agent may be used, as necessary. Examples of such alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in the form of aqueous solutions to facilitate the neutralization operation. The above-mentioned alkaline neutralizing agents may be used alone or in combination of two or more.

The degree of neutralization of the water-soluble ethylenically unsaturated monomer with an alkaline neutralizing agent, calculated as the degree of neutralization with respect to all acid groups of the water-soluble ethylenically unsaturated monomer, is preferably 10 to 100 mol%, more preferably 30 to 90 mol%, still more preferably 40 to 85 mol%, and further preferably 50 to 80 mol%.

[Polymerization initiator]

The polymerization initiator to be mixed during the polymerization step comprises an azo compound and a peroxide. These are radical polymerization initiators.

Examples of the peroxide include persulfate salts such as potassium persulfate, ammonium persulfate, and sodium persulfate, and peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide. Among these peroxides, potassium persulfate, ammonium persulfate, and sodium persulfate are preferable from the viewpoint of easy availability and easy handling.

Examples of the azo compound include 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl] propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Among these azo compounds, 2,2'-azobis(2-amidinopropane) dihydrochloride is preferable from the viewpoint of easy availability and easy handling.

The polymerization initiator may also be used as a redox polymerization initiator in combination with a reducing agent such as sodium sulfite, sodium hydrogensulfite, ferrous sulfate, or L-ascorbic acid.

From the viewpoint of more suitably exhibiting the effect of the present invention, the molar ratio of the peroxide to the azo compound (peroxide/azo compound) is preferably in the range of 0.1 to 1.0, more preferably in the range of 0.2 to 0.8, and still more preferably in the range of 0.3 to 0.6.

The total amount of the polymerization initiator to be used is, for example, 0.00005 to 0.01 mol per mole of the water-soluble ethylenically unsaturated monomer. When such a use amount is satisfied, the occurrence of an abrupt polymerization reaction can be avoided, and the polymerization reaction can be completed in an appropriate time.

[Internal crosslinking agent]

Examples of the internal crosslinking agent include those that can crosslink the polymer of the water-soluble ethylenically unsaturated monomer to be used, for example, unsaturated polyesters obtained by reacting polyols such as diols and triols, e.g., (poly)ethylene glycol, (poly)propylene glycol, 1,4-butanediol, trimethylolpropane, and (poly)glycerin, with unsaturated acids such as (meth)acrylic acid, maleic acid, and fumaric acid; bisacrylamides such as N,N-methylenebisacrylamide; di or tri(meth)acrylic acid esters obtained by reacting polyepoxides with (meth)acrylic acid; carbamyl di(meth)acrylates obtained by reacting polyisocyanates such as tolylene diisocyanate and hexamethylene diisocyanate with hydroxyethyl (meth)acrylate; compounds having two or more polymerizable unsaturated groups such as allylated starch, allylated cellulose, diallyl phthalate, N,N',N"-triallylisocyanurate, and divinylbenzene; polyglycidyl compounds such as diglycidyl compounds and triglycidyl compounds, e.g., (poly)ethylene glycol diglycidyl ether, (poly)propylene glycol diglycidyl ether, and (poly)glycerin diglycidyl ether; epihalohydrin compounds such as epichlorohydrin, epibromohydrin, and α-methylepichlorohydrin; compounds having two or more reactive functional groups such as isocyanate compounds, e.g., 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal crosslinking agents, unsaturated polyesters or polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal crosslinking agents may be used alone or in combination of two or more.

In the present invention, the molar ratio of the internal crosslinking agent to the azo compound (internal crosslinking agent/azo compound) is set within the range of 0.05 to 0.10. From the viewpoint of more suitably exhibiting the effect of the present invention, the molar ratio is preferably in the range of 0.055 to 0.095, more preferably in the range of 0.06 to 0.09, and still more preferably in the range of 0.065 to 0.085.

The amount of the internal crosslinking agent to be used is preferably 0.02 mol or less, more preferably 0.000001 to 0.01 mol, still more preferably 0.00001 to 0.005 mol, and further preferably 0.00005 to 0.002 mol, per mole of the water-soluble ethylenically unsaturated monomer.

[Hydrocarbon dispersion medium]

Examples of the hydrocarbon dispersion medium include aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are suitably used especially in terms of being industrially readily available, stable in quality, and inexpensive. These hydrocarbon dispersion media may be used alone or in combination of two or more. As examples of mixtures of hydrocarbon dispersion media, the use of commercially available products such as Exxsol Heptane (produced by ExxonMobil Corporation; containing 75 to 85% by mass of heptane and its isomeric hydrocarbons) also leads to favorable results.

The amount of the hydrocarbon dispersion medium to be used is preferably 100 to 1500 parts by mass, and more preferably 200 to 1400 parts by mass per 100 parts by mass of the first-stage water-soluble ethylenically unsaturated monomer from the viewpoint of uniformly dispersing the water-soluble ethylenically unsaturated monomer, and facilitating control of the polymerization temperature. As described below, reversed phase suspension polymerization is carried out in a single stage or two or more multiple stages. The first-stage polymerization as mentioned above refers to the first-stage polymerization reaction in single-stage polymerization or multi-stage polymerization (the same applies hereinbelow).

[Dispersion stabilizer]

(Surfactant)

In the reversed phase suspension polymerization, a dispersion stabilizer is used to improve the dispersion stability of the water-soluble ethylenically unsaturated monomer in the hydrocarbon dispersion medium. As the dispersion stabilizer, a surfactant may be used.

Examples of surfactants which can be used include sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, it is particularly preferable to use a sorbitan fatty acid ester, a polyglycerin fatty acid ester, or a sucrose fatty acid ester from the viewpoint of dispersion stability of the monomer. These surfactants may be used alone or in combination of two or more.

The amount of the surfactant to be used is preferably 0.1 to 30 parts by mass, and more preferably 0.3 to 20 parts by mass per 100 parts by mass of the first-stage water-soluble ethylenically unsaturated monomer.

(Polymeric dispersion agent)

As the dispersion stabilizer to be used in the reversed phase suspension polymerization, a polymeric dispersion agent may be used together with the surfactant described above.

Examples of the polymeric dispersion agent include maleic anhydride-modified polyethylene, maleic anhydride-modified polypropylene, maleic anhydride-modified ethylene-propylene copolymers, maleic anhydride-modified EPDM (ethylene-propylene-diene terpolymers), maleic anhydride-modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride-modified polyethylene, maleic anhydride-modified polypropylene, maleic anhydride-modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, from the viewpoint of dispersion stability of the monomer. These polymeric dispersion agents may be used alone or in combination of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

[Other components]

In the method for producing a water-absorbent resin, reversed phase suspension polymerization may be performed, if desired, with the addition of other components to the aqueous solution of the water-soluble ethylenically unsaturated monomer. As such other components, various additives such as a thickener and a chain transfer agent may be added.

As one example, reversed phase suspension polymerization may be performed with the addition of a thickener to the aqueous solution containing the water-soluble ethylenically unsaturated monomer. Adjusting the viscosity of the aqueous solution by adding a thickener in this manner makes it possible to control the median particle diameter attained in the reversed phase suspension polymerization.

As the thickener, for example, hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide can be used. With a fixed stirring speed during the polymerization, the higher the viscosity of the aqueous solution of the water-soluble ethylenically unsaturated monomer, the larger primary particles and/or secondary particles of the resulting particles tend to be.

[Reversed phase suspension polymerization]

In performing the reversed phase suspension polymerization, for example, an aqueous monomer solution containing a water-soluble ethylenically unsaturated monomer is dispersed in a hydrocarbon dispersion medium in the presence of a dispersion stabilizer. In this case, the timing of the addition of the dispersion stabilizer (surfactant or polymeric dispersion agent) may be either before or after the addition of the aqueous monomer solution as long as it is before starting the polymerization reaction.

In particular, from the viewpoint of easily reducing the amount of the hydrocarbon dispersion medium remaining in the resulting water-absorbent resin, it is preferable to perform polymerization after dispersing the aqueous monomer solution in the hydrocarbon dispersion medium in which the polymeric dispersion agent is dispersed and then further dispersing the surfactant.

Such reversed phase suspension polymerization can be carried out in one stage or in two or more multiple stages. From the viewpoint of enhancing productivity, it is preferable to perform the polymerization in two or three stages.

When the reversed phase suspension polymerization is performed in two or more multiple stages, it can be carried out by performing the first-stage reversed phase suspension polymerization, subsequently adding a water-soluble ethylenically unsaturated monomer to the reaction mixture obtained through the first-stage polymerization reaction and mixing the resulting mixture, and then performing the reversed phase suspension polymerization in the second and subsequent stages in the same manner as in the first stage. In the reversed phase suspension polymerization in each of the second and subsequent stages, it is preferable that reversed phase suspension polymerization is performed with addition of a polymerization initiator and, as necessary, an internal crosslinking agent in addition to a water-soluble ethylenically unsaturated monomer in amounts within the above-described molar ratio ranges of the respective components with respect to the water-soluble ethylenically unsaturated monomer based on the amount of the water-soluble ethylenically unsaturated monomer to be added at the time of the reversed phase suspension polymerization in each of the second and subsequent stages. The amount of the water-soluble ethylenically unsaturated monomer and the ratios of the polymerization initiator, the internal crosslinking agent, and the like to the water-soluble ethylenically unsaturated monomer may be the same or different among the first stage and the second and subsequent stages as long as they are within the above ranges.

In addition, in the case of adopting the polymerization step in which reversed phase suspension polymerization is performed in two or more multiple stages, it is preferable that in at least the first stage polymerization step, the concentration of the water-soluble ethylenically unsaturated monomer in the aqueous solution containing the water-soluble ethylenically unsaturated monomer is set to 40% by mass or more and the molar ratio of the azo compound of the polymerization initiator and the internal crosslinking agent (internal crosslinking agent/azo compound) is set within the range of 0.05 to 0.10, and it is more preferable that also in the reversed phase suspension polymerization in each of the second and subsequent stages, the concentration and the molar ratio are set within those ranges. The concentration of the water-soluble ethylenically unsaturated monomer and the molar ratio of the polymerization initiator (internal crosslinking agent/azo compound) may be the same or different among the first stage and the second and subsequent stages as long as they are within the above ranges.

The reaction temperature of the polymerization reaction is preferably 20 to 110°C, and more preferably 40 to 90°C from the viewpoint of allowing the polymerization to proceed quickly to reduce the polymerization time for improved economic efficiency, and readily removing the heat of polymerization to perform a smooth reaction.

<Post-crosslinking step>

The post-crosslinking step is a step of post-crosslinking a polymer (hydrous gel having an internally crosslinked structure) obtained by polymerizing a water-soluble ethylenically unsaturated monomer in the polymerization step. The water-absorbent resin of the present invention is obtained by mixing the polymer obtained in the polymerization step with a post-crosslinking agent to crosslink the polymer (post-crosslinking reaction). This post-crosslinking reaction is performed in the presence of a post-crosslinking agent after polymerization of a water-soluble ethylenically unsaturated monomer. By performing such a post-crosslinking reaction, it is possible to obtain a water-absorbent resin having various improved performances such as water retention capacity, water absorption capacity under a load, and non-pressurization DW.

From the viewpoint of more suitably exhibiting the effect of the present invention, in the post-crosslinking step, the molar ratio of the post-crosslinking agent to the water-soluble ethylenically unsaturated monomer (post-crosslinking agent/water-soluble ethylenically unsaturated monomer) is preferably in the range of 0.00001 to 0.001, more preferably in the range of 0.00005 to 0.0005, and still more preferably in the range of 0.0001 to 0.0003.

Examples of the post-crosslinking agent include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and α-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylenebisoxazoline; carbonate compounds such as ethylene carbonate; and hydroxyalkylamide compounds such as bis[N,N-di(β-hydroxyethyl)]adipamide. Among these post-crosslinking agents, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferred. These post-crosslinking agents may be used alone or in combination of two or more.

The amount of the post-crosslinking agent to be used is preferably 0.00001 to 0.01 mol, more preferably 0.00005 to 0.005 mol, and still more preferably 0.0001 to 0.002 mol per mole of all the water-soluble ethylenically unsaturated monomer used in the polymerization.

As a method of adding the post-crosslinking agent, the post-crosslinking agent may be added either as it is or in the form of an aqueous solution, and as necessary, it may be added in the form of a solution prepared using a hydrophilic organic solvent as a solvent. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used alone, in combination of two or more, or in the form of a mixed solvent with water.

The timing of the addition of the post-crosslinking agent may be any time after the polymerization of the water-soluble ethylenically unsaturated monomer, and it is preferable to add the post-crosslinking agent in the presence of 1 to 400 parts by mass of water per 100 parts by mass of the water-soluble ethylenically unsaturated monomer, more preferably added in the presence of 5 to 200 parts by mass of water, still more preferably added in the presence of 10 to 100 parts by mass of water, and even more preferably added in the presence of 20 to 60 parts by mass of water. The amount of water herein refers to the total amount of the water contained in the reaction system and the water that is used, as required, during the addition of the post-crosslinking agent.

The reaction temperature during the post-crosslinking reaction is preferably 50 to 250°C, more preferably 60 to 180°C, still more preferably 60 to 140°C, and further preferably 70 to 120°C. The reaction time of the post-crosslinking reaction is preferably 1 to 300 minutes, and more preferably 5 to 200 minutes.

<Drying step>

The method may include a drying step of removing water, a hydrocarbon dispersion medium, and the like by distillation by externally applying energy such as heat after performing the reversed phase suspension polymerization described above. When dehydration from the hydrous gel after the reversed phase suspension polymerization is performed, the system in which the hydrous gel is dispersed in the hydrocarbon dispersion medium is heated, so that water and the hydrocarbon dispersion medium are temporarily distilled off to the outside of the system by azeotropic distillation. At this time, returning only the removed hydrocarbon dispersion medium into the system permits continuous azeotropic distillation. This embodiment is preferable from the viewpoint that the resin is hardly degraded because the temperature in the system during drying is maintained at or below the azeotropic temperature with the hydrocarbon dispersion medium. Subsequently, water and the hydrocarbon dispersion medium are distilled off, and thus a water-absorbent resin is obtained. By controlling the processing conditions in this drying step after the polymerization and thereby adjusting the amount of dehydration, it is possible to control various performances of the resulting water-absorbent resin.

In the drying step, the drying treatment by distillation may be performed either under normal pressure or under reduced pressure. From the viewpoint of increasing the drying efficiency, the drying treatment may be performed under a flow of gas such as nitrogen. When the drying treatment is performed under normal pressure, the drying temperature is preferably 70 to 250°C, more preferably 80 to 180°C, still more preferably 80 to 140°C, and further preferably 90 to 130°C. In the case where the drying treatment is performed under reduced pressure, the drying temperature is preferably 40 to 160°C, and more preferably 50 to 110°C.

When the post-crosslinking step using the post-crosslinking agent is performed, the above-described drying step by distillation is performed after the completion of the post-crosslinking step. Alternatively, the post-crosslinking step and the drying step may be performed simultaneously.

The water-absorbent resin of the present invention may contain an additive according to the purpose. Examples of such additives include inorganic powders, surfactants, oxidants, reducing agents, metal chelating agents, radical chain inhibitors, antioxidants, and antibacterial agents. For example, by adding 0.05 to 5 parts by mass of amorphous silica as an inorganic powder to 100 parts by mass of the water-absorbent resin, the flowability of the water-absorbent resin can be further improved.

2. Water-absorbent resin

By employing the method for producing a water-absorbent resin of the present invention, for example, a water-absorbent resin having the following properties (A) to (C) can be suitably produced.

(A) The total value (X + Y) of the physiological saline retention capacity X and the water absorption capacity Y under a load of 4.14 kPa is 60 or more.
(B) The total value (X + Z) of the physiological saline retention capacity X and the value Z of the non-pressurization DW (Demand Wettability) after 5 minutes is 90 or more.
(C) The physiological saline retention capacity X is 44 g/g or more and 70 g/g or less.

By employing the method for producing a water-absorbent resin of the present invention, it is also possible to suitably produce a water-absorbent resin that is a crosslinked product of a polymer of a water-soluble ethylenically unsaturated monomer, in which the water-soluble ethylenically unsaturated monomer has a content of acrylic acid and a salt thereof of 70 mol% or more and 100 mol% or less, and that has the following properties (A) and (B).

(A) The total value (X + Y) of the physiological saline retention capacity X and the water absorption capacity Y under a load of 4.14 kPa is 60 or more.
(B) The total value (X + Z) of the physiological saline retention capacity X and the value Z of the non-pressurization DW after 5 minutes is 90 or more.

The total value (X + Y) of (A) of the water-absorbent resin of the present invention is preferably 62 or more, more preferably 64 or more, and still more preferably 66 or more, and is preferably 100 or less, more preferably 85 or less, and still more preferably 75 or less.

The total value (X + Z) of (B) of the water-absorbent resin of the present invention is preferably 95 or more, more preferably 100 or more, and still more preferably 105 or more, and is preferably 150 or less, more preferably 130 or less, and still more preferably 120 or less.

The physiological saline retention capacity X of the water-absorbent resin of the present invention is preferably 46 g/g or more, more preferably 49 g/g or more, and still more preferably 52 g/g or more, and is preferably 70 g/g or less, more preferably 65 g/g or less, and still more preferably 60 g/g or less.

The water absorption capacity Y of the water-absorbent resin of the present invention under a load of 4.14 kPa is preferably 12 ml/g or more, more preferably 14 ml/g or more, and still more preferably 16 ml/g or more, and is preferably 33 ml/g or less, more preferably 27 ml/g or less, and still more preferably 23 ml/g or less.

The value Z of non-pressurization DW after 5 minutes of the water-absorbent resin of the present invention is preferably 42 ml/g or more, more preferably 48 ml/g or more, and still more preferably 50 ml/g or more, and is preferably 80 ml/g or less, more preferably 70 ml/g or less, and still more preferably 65 ml/g or less.

With the water-absorbent resin of the present invention, the number of the addition of a liquid until the occurrence of leakage measured by a slope absorption test is preferably 4 or more. The number of addition is, for example, 7 times or less. The amount of the liquid absorbed until the occurrence of leakage is preferably 240 g or more, and more preferably 270 g or more. The amount of the liquid absorbed before the occurrence of leakage is, for example, 550 g or less, 520 g or less, or 490 g or less.

The methods for measuring the physiological saline retention capacity X, the water absorption capacity Y under a load of 4.14 kPa, and the value Z of non-pressurization DW after 5 minutes of the water-absorbent resin, and the method of the slope absorption test are as described in EXAMPLE, respectively.

The water-absorbent resin of the present invention is constituted of a product obtained by crosslinking a polymer of a water-soluble ethylenically unsaturated monomer, namely, a crosslinked polymer having a structural unit derived from a water-soluble ethylenically unsaturated monomer.

The water-absorbent resin may have various shapes. Examples of the shape of the water-absorbent resin include a granular shape, a substantially spherical shape, an indefinitely crushed shape, a plate shape, a fibrous shape, a flake shape, and a shape in which these resins are aggregated. The water-absorbent resin preferably has a granular shape, a substantially spherical shape, an indefinitely crushed shape, a fibrous shape, a shape in which these resins are aggregated, or the like.

The water-absorbent resin not only may be in a form in which each particle is composed of a single particle, but also may be in a form (secondary particle) in which fine particles (primary particles) are aggregated. Examples of the shape of the primary particles include a substantially spherical shape, an indefinitely crushed shape, and a plate shape. Examples of the primary particles produced by reversed phase suspension polymerization include substantially spherical single particles having a smooth surface shape such as a perfect spherical shape and an ellipsoidal shape.

The primary particles having such a shape and having a smooth surface shape thus have high flowability as a powder, and aggregated particles are easily densely packed and thus are less prone to be broken even when subjected to impact, so that a water-absorbent resin having high particle strength is provided.

The median particle diameter of the water-absorbent resin is preferably 200 $\mu$m or more, 250 $\mu$m or more, 280 $\mu$m or more, 300 $\mu$m or more, or 320 $\mu$m or more from the viewpoint of more suitably exhibiting the effect of the present invention. From the same point of view, the median particle diameter is preferably 700 $\mu$m or less, 600 $\mu$m or less, 550 $\mu$m or less, 500 $\mu$m or less, 450 $\mu$m or less, or 400 $\mu$m or less. That is, the median particle diameter is preferably 200 to 700 $\mu$m, preferably 200 to 600 $\mu$m, more preferably 250 to 500 $\mu$m, still more preferably 300 to 450 $\mu$m, and further preferably 320 to 400 $\mu$m.

The median particle diameter of the particulate water-absorbent resin can be measured using JIS standard sieves, and specifically, is a value measured by the method described in EXAMPLES.

3. Absorber, absorbent article

The water-absorbent resin of the present invention constitutes an absorber to be used for sanitary materials such as feminine hygiene products and disposable diapers, and is suitably used for an absorbent article including the absorber.

The absorber of the present invention includes the water-absorbent resin of the present invention. The absorber may further include hydrophilic fibers. Examples of the configuration of the absorber include a sheet-like structure in which the water-absorbent resin is fixed on a nonwoven fabric or between a plurality of nonwoven fabrics, a mixed dispersion obtained by mixing the water-absorbent resin and hydrophilic fibers to attain a uniform composition, a sandwich structure in which the water-absorbent resin is sandwiched between layered hydrophilic fibers, and a structure in which the water-absorbent resin and hydrophilic fibers are wrapped with tissue. The absorber may contain other components, for example, an adhesive binder such as a thermally fusible synthetic fiber, a hot-melt adhesive, or an adhesive emulsion for enhancing the shape retention of the absorber.

The basis weight of the water-absorbent resin in the absorber of the present invention is 50 g/m$^2$ or more and 400 g/m$^2$ or less. From the viewpoint of more suitably exhibiting the effect of the present invention, the basis weight is preferably 100 g/m$^2$ or more, more preferably 120 g/m$^2$ or more, and still more preferably 140 g/m$^2$ or more, and is preferably 300 g/m$^2$ or less, more preferably 250 g/m$^2$ or less, and still more preferably 200 g/m$^2$ or less.

The content of the water-absorbent resin in the absorber is preferably 5 to 100% by mass, more preferably 10 to 95% by mass, still more preferably 20 to 90% by mass, and further preferably 30 to 80% by mass.

The hydrophilic fiber may be at least one selected from the group consisting of finely pulverized wood pulp, cotton, cotton linters, rayon, cellulose acetates, polyamides, polyesters, and polyolefins. Examples thereof include cellulose fibers obtained from wood such as fluff pulp, mechanical pulp, chemical pulp, and semi-chemical pulp; artificial cellulose fibers such as rayon and acetate, and fibers made of synthetic resin, such as polyamide, polyester, or polyolefin, subjected to hydrophilization treatment. The average fiber length of the hydrophilic fibers is usually 0.1 to 10 mm, or may be 0.5 to 5 mm.

The absorber using the water-absorbent resin of the present invention can be held between a liquid-permeable sheet (top sheet) through which a liquid can pass and a liquid-impermeable sheet (back sheet) through which a liquid cannot pass to form the absorbent article of the present invention. The liquid-permeable sheet is disposed on the side where the absorber comes into contact with the body, and the liquid-impermeable sheet is disposed opposite from the side where the absorber comes into contact with the body.

Examples of the liquid-permeable sheet include nonwoven fabrics of an air-through type, a spunbond type, a chemical bond type, a needle punch type, or the like made of fibers of polyethylene, polypropylene, polyester, or the like, and porous synthetic resin sheets. Examples of the liquid-impermeable sheet include synthetic resin films made of resins such as polyethylene, polypropylene, and polyvinyl chloride. The liquid-permeable sheet is preferably at least one selected from the group consisting of a thermally bonded nonwoven fabric, an air-through nonwoven fabric, a spunbond nonwoven fabric, and a spunbond/melt-blown/spunbond nonwoven fabric.

The basis weight of the liquid-permeable sheet is preferably 5 g/m$^2$ or more and 100 g/m$^2$ or less, and more preferably 10 g/m$^2$ or more and 60 g/m$^2$ or less. The surface of the liquid-permeable sheet may be embossed or perforated in order to improve the liquid diffusibility. The embossing and the perforating can be performed by known methods.

Examples of the liquid-impermeable sheet include a sheet made of a synthetic resin such as polyethylene, polypropylene, or polyvinyl chloride, a sheet made of a nonwoven fabric such as spunbond/melt-blown/spunbond (SMS) nonwoven fabric in which a water-resistant melt-blown nonwoven fabric is sandwiched between high-strength spunbond nonwoven fabrics, and a sheet made of a composite material of these synthetic resins and a nonwoven fabric (for example, a spunbond nonwoven fabric or a spunlace nonwoven fabric). As the liquid-impermeable sheet, a sheet made of a synthetic resin mainly containing a low-density polyethylene (LDPE) resin can also be used. The liquid-impermeable sheet may be, for example, a sheet made of a synthetic resin having a basis weight of 10 to 50 g/m$^2$.

The absorbent article preferably includes a laminate including an absorber containing a water-absorbent resin, and core wraps sandwiching the absorbent from upper and lower sides of the absorbent; a liquid-permeable sheet disposed on an upper surface of the laminate; and a liquid-impermeable sheet disposed on a surface of the laminate on a side opposite from the liquid-permeable sheet.

EXAMPLES

The present invention will be hereinafter described in detail by way of examples and comparative examples. It, however, is to be noted that the present invention is not limited to the examples.

<Production of water-absorbent resin>

(Example 1)

A 2-L cylindrical round-bottomed separable flask having an inside diameter of 11 cm and equipped with a reflux condenser, a dropping funnel, a nitrogen gas inlet tube, and a stirrer having stirring blades composed of two sets of four inclined paddle blades with a blade diameter of 5 cm was prepared. This flask was charged with 252 g of n-heptane as a hydrocarbon dispersion medium, followed by addition of 0.736 g of a maleic anhydride-modified ethylene-propylene copolymer (Hi-WAX 1105A available from Mitsui Chemicals, Inc.) as a polymeric dispersion agent, and then the temperature was raised to 80°C with stirring to dissolve the dispersion agent, and then cooled to 50°C.

Meanwhile, in a 300-ml beaker was placed 92.0 g (1.03 mol) of an 80.5% by mass aqueous acrylic acid solution as an ethylenically unsaturated monomer, and 102.78 g of a 30% by mass aqueous sodium hydroxide solution was added dropwise with external cooling to perform 75 mol% neutralization. Then, 0.0920 g of hydroxyethylcellulose (HEC AW-15F available from Sumitomo Seika Chemicals Co. Ltd.) as a thickener, 0.0920 g (0.339 mmol) of 2,2'-azobis(2-amidinopropane) dihydrochloride as an azo compound and 0.0276 g (0.102 mmol) of potassium persulfate as a peroxide, 0.00460 g (0.0264 mmol) of ethylene glycol diglycidyl ether as an internal crosslinking agent, and 8.28 g of ion-exchanged water were added and dissolved. As a result, a first-stage aqueous monomer solution was prepared.

The prepared first-stage aqueous monomer solution was added to the reaction liquid in the separable flask and stirred for 10 minutes. Next, a surfactant solution prepared by dissolving 0.736 g of sucrose stearate (HLB: 3, Mitsubishi-Kagaku Foods Corporation, Ryoto Sugar Ester S-370) as a surfactant in 6.62 g of n-heptane by heating was further added to the reaction solution, and the inside of the system was sufficiently replaced with nitrogen while stirring at a rotation speed of the stirrer of 600 rpm. Thereafter, the flask was soaked in a water bath at 70°C to raise the temperature, and polymerization was performed for 60 minutes. Thereby, a first-stage polymerization slurry was obtained.

Next, 128.8 g (1.44 mol) of an 80.5% by mass aqueous solution of acrylic acid as an ethylenically unsaturated monomer was placed in another beaker having an internal volume of 500 ml, and 143.89 g of a 30% by mass aqueous solution of sodium hydroxide was added dropwise thereto with external cooling to perform 75 mol% neutralization. Thereafter, 0.129 g (0.475 mmol) of 2,2'-azobis(2-amidinopropane) dihydrochloride as an azo compound, 0.0386 g (0.143 mmol) of potassium persulfate as a peroxide, 0.0116 g (0.0665 mmol) of ethylene glycol diglycidyl ether as an internal crosslinking agent, and 11.2 g of ion-exchanged water were added thereto and dissolved. Thereby, a second-stage aqueous monomer solution was prepared.

The inside of the separable flask system was cooled to 25°C with stirring at a rotation speed of the stirrer of 1000 rpm. Then, the whole amount of the second-stage aqueous monomer solution was added to the first-stage polymerization slurry in the separable flask, and the inside of the system was replaced with nitrogen for 30 minutes. Thereafter, the flask was soaked in the water bath at 70°C again to raise the temperature, and a polymerization reaction was performed for 60 minutes.

To the reaction solution containing hydrous gel polymer resulting from the second-stage polymerization was added with stirring 0.265 g of a 45% by mass aqueous solution of pentasodium diethylenetriamine pentaacetate. Then, the flask was soaked in an oil bath set at 125°C, and 219.3 g of water was removed out of the system by azeotropic distillation of water and n-heptane. Then, 4.42 g (0.507 mmol) of a 2% by mass aqueous ethylene glycol diglycidyl ether solution was added to the flask, and the mixture was kept at 83°C for 2 hours.

Thereafter, the resulting mixture was dried by evaporating n-heptane at 125°C, affording polymer particles (dry product). The polymer particles were passed through a sieve with a mesh size of 850 $\mu$m, and 0.2% by mass, based on the mass of the polymer particles, of amorphous silica (available from Oriental Silicas Corporation, TOKUSIL NP-S, hydrophilic) was mixed with the polymer particles, affording 225.1 g of a water-absorbent resin containing the amorphous silica. The median particle diameter of the water-absorbent resin was 336 $\mu$m.

(Example 2)

220.2 g of a water-absorbent resin was obtained in the same manner as in Example 1 except that the amount of water to be removed out of the system by azeotropic distillation was changed to 223.9 g. The median particle diameter of the water-absorbent resin was 350 $\mu$m.

(Example 3)

225.3 g of a water-absorbent resin was obtained in the same manner as in Example 1 except that the amount of water to be removed out of the system by azeotropic distillation was changed to 225.8 g. The median particle diameter of the water-absorbent resin was 354 $\mu$m.

(Example 4)

203.7 g of a water-absorbent resin was obtained in the same manner as in Example 1 except that the addition amount of the azo compound 2,2'-azobis(2-amidinopropane) dihydrochloride to be dissolved in the first-stage aqueous monomer solution was changed to 0.0460 g (0.170 mmol), the addition amount of the internal crosslinking agent ethylene glycol diglycidyl ether was changed to 0.00184 g (0.0106 mmol), the amount of ion-exchanged water was changed to 9.47 g, and the amount of water to be removed out of the system by azeotropic distillation was changed to 219.8 g. The median particle diameter of the water-absorbent resin was 353 $\mu$m.

(Example 5)

216.4 g of a water-absorbent resin was obtained in the same manner as in Example 4 except that the amount of water to be removed out of the system by azeotropic distillation was changed to 221.8 g. The median particle diameter of the water-absorbent resin was 334 $\mu$m.

(Comparative Example 1)

229.0 g of a water-absorbent resin was obtained in the same manner as in Example 1 except that the amount of n-heptane to be used as a hydrocarbon dispersion medium was changed to 293 g, the amount of ion-exchanged water to be added to the first-stage aqueous monomer solution was changed to 40.93 g, the rotation speed in the first-stage polymerization was changed to 550 rpm, and the amount of water to be removed out of the system by azeotropic distillation was changed to 236.0 g. The median particle diameter of the water-absorbent resin was 348 $\mu$m.

(Comparative Example 2)

229.0 g of a water-absorbent resin was obtained in the same manner as in Comparative Example 1 except that the amount of water to be removed out of the system by azeotropic distillation was changed to 245.2 g. The median particle diameter of the water-absorbent resin was 348 m.

(Comparative Example 3)

201.0 g of a water-absorbent resin was obtained in the same manner as in Example 1 except that the addition amount of the internal crosslinking agent ethylene glycol diglycidyl ether to be dissolved in the first-stage aqueous monomer solution was changed to 0.00184 g (0.0106 mmol), the amount of ion-exchanged water was changed to 8.55 g, and the amount of water to be removed out of the system by azeotropic distillation was changed to 210.1 g. The median particle diameter of the water-absorbent resin was 403 $\mu$m.

(Comparative Example 4)

216.8 g of a water-absorbent resin was obtained in the same manner as in Comparative Example 3 except that the amount of water to be removed out of the system by azeotropic distillation was changed to 213.7 g. The median particle diameter of the water-absorbent resin was 353 $\mu$m.

(Comparative Example 5)

217 g of a water-absorbent resin was obtained in the same manner as in Example 1 except that the addition amount of the azo compound 2,2'-azobis(2-amidinopropane) dihydrochloride to be dissolved in the first-stage aqueous monomer solution was changed to 0.0460 g (0.170 mmol), the amount of ion-exchanged water was changed to 9.20 g, and the amount of water to be removed out of the system by azeotropic distillation was changed to 227.2 g. The median particle diameter of the water-absorbent resin was 403 $\mu$m.

(Comparative Example 6)

229.0 g of a water-absorbent resin was obtained in the same manner as in Example 1 except that the amount of n-heptane to be used as a hydrocarbon dispersion medium was changed to 293 g, 2,2'-azobis(2-amidinopropane) dihydrochloride, an azo compound, to be dissolved in the first-stage aqueous monomer solution was not added, the amount of potassium persulfate, a peroxide, was changed to 0.0736 g (0.272 mmol), the addition amount of ethylene glycol diglycidyl ether, an internal crosslinking agent, was changed to 0.00276 g (0.0158 mmol), the amount of ion-exchanged water was changed to 40.7 g, the rotation speed during the first-stage polymerization was changed to 550 rpm, 2,2'-azobis(2-amidinopropane) dihydrochloride, an azo compound, to be dissolved in the second-stage aqueous monomer solution was not added, the amount of potassium persulfate, a peroxide, was changed to 0.103 g (0.381 mmol), the amount of ion-exchanged water was changed to 10.6 g, and the amount of water to be removed out of the system by azeotropic distillation was changed to 266.4 g. The median particle diameter of the water-absorbent resin was 348 $\mu$m.

The properties of the water-absorbent resins obtained in Examples and Comparative Examples were evaluated by the following tests. Unless otherwise specified, the measurement was performed under an environment of a temperature of 25 $\pm$ 2°C and a humidity of 50 $\pm$ 10%. Hereinafter, each evaluation test method will be described.

<Physiological saline retention capacity>

A cotton bag (Cottonbroad No. 60, 100 mm in width, 200 mm in length) in which 2.0 g of the water-absorbent resin particles had been weighed was placed in a 500-mL beaker. 500 g of a 0.9% by mass aqueous sodium chloride solution (physiological saline) was poured into the cotton bag containing the water-absorbent resin at a time such that no lumps were formed. Then, the top of the cotton bag was tied with a rubber band and the resultant was left at rest for 30 minutes, and thereby the water-absorbent resin was swollen. The cotton bag after 30 minutes lapse was dehydrated for one minute using a dehydrator (product number: H-122, manufactured by KOKUSAN Co., Ltd.) set at a centrifugal force of 167 G, and the mass Wa (g) of the dehydrated cotton bag containing the swollen gel was measured. The same operation was performed without adding a water-absorbent resin, and the empty mass Wb (g) of the cotton bag in a wet state was measured, and the physiological saline retention capacity was calculated from the following formula.

$$\text{Physiological saline retention capacity [g/g]} = [\text{Wa} - \text{Wb}]/2.0$$

<Physiological saline absorption capacity under a load of 4.14 kPa>

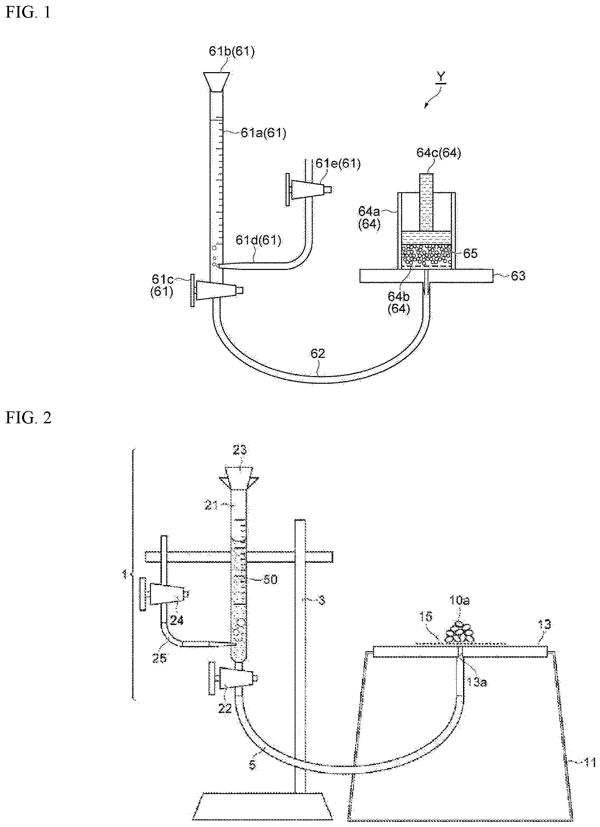

The physiological saline absorption capacity (room temperature, 25°C $\pm$ 2°C) under a load (under pressurization) of a water-absorbent resin was measured using a measuring device Y illustrated in Fig. 1. The measuring device Y is composed of a burette part 61, a conduit pipe 62, a measuring stand 63, and a measuring part 64 placed on the measuring stand 63. The burette part 61 includes a burette 61a extending in a vertical direction, a rubber stopper 61b disposed at an upper end of the burette 61a, a cock 61c disposed at a lower end of the burette 61a, an air introducing pipe 61d having one end extending to the burette 61a in the vicinity of the cock 61c, and a cock 61e disposed at the other end side of the air introducing pipe 61d. The conduit pipe 62 is attached between the burette part 61 and the measuring stand 63. The conduit pipe 62 has an inner diameter of 6 mm. There is a hole having a diameter of 2 mm in a center portion of the measuring stand 63, and the conduit pipe 62 is connected to the hole. The measuring part 64 includes a cylinder 64a (made of acrylic resin (PLEXIGLAS)), a nylon mesh 64b adhered to a bottom portion of the cylinder 64a, and a weight 64c. The cylinder 64a has an inner diameter of 20 mm. The nylon mesh 64b has a mesh size of 75 $\mu$m (200 mesh). At the time of measurement, a water-absorbent resin 65 to be measured is uniformly scattered on the nylon mesh 64b. The weight 64c has a diameter of 19 mm, and the weight 64c has a mass of 120 g. The weight 64c is placed on the water-absorbent resin 65, and a load of 4.14 kPa can be applied to the water-absorbent resin 65.

0.100 g of the water-absorbent resin 65 was put into the cylinder 64a of the measuring device Y, then the weight 64c was placed thereon and the measurement was started. Because air of the same volume as that of the physiological saline absorbed by the water-absorbent resin 65 is quickly and smoothly supplied into the burette 61a through the air introducing pipe, a reduced amount in water level of the physiological saline in the burette 61a indicates an amount of the physiological saline absorbed by the water-absorbent resin 65. A scale of the burette 61a was engraved from 0 mL in increments of 0.5 mL from the top to bottom direction. A scale Va of the burette 61a before the start of water absorption and a scale Vb of the burette 61a 60 minutes after the start of water absorption were read as water levels of the physiological saline, and a physiological saline absorption capacity under a load of 4.14 kPa was calculated from the following formula.

$$\text{Physiological saline absorption capacity [mL/g] under a load of } 4.14 \text{ kPa} = (\text{Vb} - \text{Va})/0.100$$

<Value of non-pressurization DW (Demand Wettability) after 5 minutes>

The non-pressurization DW of particles of a water-absorbent resin was measured using a measuring device illustrated in Fig. 2. The measurement was carried out five times for one water-absorbent resin, and an average value of three measured values excluding a minimum value and a maximum value was determined. The measuring device has a burette part 1, a conduit pipe 5, a measuring stand 13, a nylon mesh sheet 15, a stand 11, and a clamp 3. The burette part 1 has a burette tube 21 on which a scale is engraved, a rubber stopper 23 for sealing an opening at an upper part of the burette tube 21, a cock 22 connected to a distal end of a lower part of the burette tube 21, an air introducing pipe 25 connected to the lower part of the burette tube 21, and a cock 24. The burette part 1 is fixed by the clamp 3. The flat plate-shaped measuring stand 13 has a through-hole 13a having a diameter of 2 mm and formed in the center portion of the measuring stand 13, and is supported by the height-variable stand 11. The through-hole 13a of the measuring stand 13 and the cock 22 of the burette part 1 are connected by the conduit pipe 5. The conduit pipe 5 has an inner diameter of 6 mm.

The measurement was performed under an environment of a temperature of 25°C and a humidity of 60 ± 10%. First, the cock 22 and the cock 24 of the burette part 1 were closed, and a 0.9% by mass saline solution 50 that had been adjusted to 25°C was put into the burette tube 21 through the opening at the upper part of the burette tube 21. The concentration of 0.9% by mass of the saline solution is a concentration based on the mass of the saline solution. The opening of the burette tube 21 was sealed with the rubber stopper 23, and then the cock 22 and the cock 24 were opened. The inside of the conduit pipe 5 was filled with the 0.9% by mass saline solution 50 so as to prevent air bubbles from entering. The height of the measuring stand 13 was adjusted such that the height of a water surface of the 0.9% by mass saline solution having reached the inside of the through-hole 13a was the same as the height of an upper surface of the measuring stand 13. After the adjustment, the height of the water surface of the 0.9% by mass saline solution 50 in the burette tube 21 was read on the scale engraved on the burette tube 21, and this position was defined as a zero point (value read at 0 seconds).

The nylon mesh sheet 15 (100 mm × 100 mm, 250 mesh, thickness: about 50 $\mu$m) was laid in the vicinity of the through-hole 13a on the measuring stand 13, and a cylinder having an inner diameter of 30 mm and a height of 20 mm was placed on the center portion of the nylon mesh sheet. 1.00 g of a water-absorbent resin 10a were uniformly scattered in the cylinder. Thereafter, the cylinder was carefully removed, affording a sample in which the water-absorbent resin 10a was dispersed in a circle shape in the center portion of the nylon mesh sheet 15. Subsequently, the nylon mesh sheet 15 on which the water-absorbent resin 10a was placed was moved rapidly to such an extent that the water-absorbent resin 10a was not dissipated such that the center of the nylon mesh sheet was located at the position of the through-hole 13a, and then the measurement was started. The time when air bubbles were first introduced from the air introducing pipe 25 into the burette tube 21 was defined as a start of water absorption (0 seconds).

A decrease amount of the 0.9% by mass saline solution 50 in the burette tube 21 (that is, an amount of the 0.9% by mass saline solution absorbed by the water-absorbent resin 10a) was sequentially read, and a decrease in weight Wc (g) of the 0.9% by mass saline solution 50 after 5 minutes from the start of the water absorption by the water-absorbent resin 10a was read. From the Wc, a value of non-pressurization DW after 5 minutes was determined by the following formula. The non-pressurization DW is a water absorption capacity per 1.00 g of the water-absorbent resin 10a.

$$\text{Value (mL/g) of non-pressurization DW} = \text{Wc}/1.00$$

<Method for measuring median particle diameter>

10 g of a granular water-absorbent resin was sieved using a continuous automated sonic sieving particle size analyzer (Robot Sifter RPS-205, manufactured by Seishin Enterprise Co., Ltd.), sieves with JIS standard mesh sizes of 850 $\mu$m, 710 $\mu$m, 600 $\mu$m, 500 $\mu$m, 425 $\mu$m, 300 $\mu$m, 250 $\mu$m, and 150 $\mu$m, and a receptacle. A mass of the particles remaining on each sieve was calculated as a mass percentage relative to the total amount. The mass percentage of the particles remaining on each sieve was integrated in a descending order of particle sizes, and the relationship between the mesh size and an integrated value of the mass percentage of particles remaining on the sieve was plotted on a logarithmic probability paper. The plotted points on the probability paper were connected by straight lines, and thereby a particle diameter corresponding to 50% by mass of the integrated mass percentage was determined and taken as a median particle diameter.

<Preparation of absorber and absorbent article>

Using the water-absorbent resins obtained in Example 2 to 5 and Comparative Examples 2 and 6, absorbers and absorbent articles were prepared by the following procedure.

(Example 6)

Using an air flow mixer (Pad former manufactured by Autec Ltd.), 9.6 g of the water-absorbent resin obtained in Example 2 and 9.6 g of pulverized pulp were uniformly mixed by air papermaking to prepare a sheet-shaped absorber having a size of 12 cm $\times$ 32 cm. The absorber was disposed on a tissue paper (core wrap) having a basis weight of 16 g/m$^2$, and a tissue paper (core wrap) was laminated on the absorber in this order. A load of 141 kPa was applied to the laminate for 30 seconds. Further, a hydrophilic air-through nonwoven fabric (Rengo Nonwoven Products Co., Ltd., basis weight: 21 g/m$^2$) having a size of 12 cm $\times$ 32 cm was laminated to prepare an absorbent article for testing. In the absorbent article, the basis weight of the water-absorbent resin was 250 g/m$^2$, and the basis weight of the pulverized pulp (hydrophilic fiber) was 250 g/m$^2$.

(Examples 7 to 9 and Comparative Examples 7 and 8)

Absorbent articles were prepared in the same manner as in Example 6 except that the water-absorbent resin was changed to the water-absorbent resins obtained in Examples 3 to 5, Comparative Example 2, and Comparative Example 6.

<Slope absorption test of absorbent article>

The slope absorption test of each water-absorbent article was performed by the following procedure.

(Preparation of test solution)

A test solution was prepared by blending and dissolving components in ion-exchanged water such that inorganic salts were present as shown below, and further blending a small amount of Blue No. 1.

Composition of test solution

- Deionized water 5919.6 g
- NaCl 60.0 g
- $CaCl_2 \cdot H_2O$ 1.8 g
- $MgCl_2 \cdot 6H_2O$ 3.6 g
- Food Blue No. 1 (for coloring)
- 1% - Triton X-100 15.0 g

(Slope absorption test)

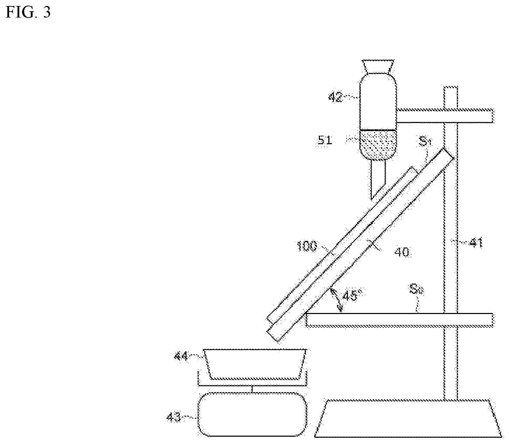

Fig. 3 is a schematic diagram illustrating a method of evaluating the leakage property of an absorbent article at the time of inclination. A support plate (an acrylic resin plate was used here; hereinafter also referred to as an inclined plane S1) with a length of 45 cm and having a flat main surface was fixed by a stand 41 in a state of being inclined at 45 $\pm$ 2 degrees with respect to a horizontal plane S0. In a room at a temperature of 25 $\pm$ 2°C, an absorbent article 100 for testing was bonded onto the inclined plane S 1 of the fixed support plate such that a longitudinal direction of the absorbent article was along a longitudinal direction of the support plate. Next, a test solution 51 adjusted to 25 $\pm$ 1°C was dropped from a dropping funnel 42 disposed vertically above the absorbent article toward a position 8 cm, in an upper direction, from the center of an absorber in the absorbent article 100. 80 mL of the test solution (density: 1.0 g/mL) was dropped per one time at a rate of 8 mL/sec. The distance between the tip of the dropping funnel 42 and the absorbent article was 10 ± 1 mm. The test solution was repeatedly added under the same conditions at intervals of 10 minutes from the start of the first addition of the test solution, and the test solution was added six times in total.

In a case where the test solution that was not absorbed by the absorbent article 100 leaked out from a lower part of the support plate, the leaked test solution was recovered in a metal tray 44 disposed below the support plate. The mass (g) of the recovered test solution was measured with a balance 43, and the value was recorded as the amount of leakage. The amount of absorption was calculated by subtracting the amount of leakage from the total amount of the test solution added (80 mL × 6 times = 480 mL). In addition, the absorption rate at the time of inclination was calculated by the following formula.

$$\text{Absorption rate at inclination (\%)} = \text{amount of absorption (g)/total amount of test solution added (g)} \times 100$$

As this numerical value is larger, it is determined that leakage of liquid is less likely to occur when the absorbent article is worn.

[Table 1]

| Table 1 | Conditions of water-absorbent resin production | | Property of water-absorbent resin | | | X + Y | X + Z |
| --- | --- | --- | --- | --- | --- | --- | --- |
| | Concentration of monomer in aqueous solution mixed in polymerization step | Internal crosslinking agent/Azo compound | Physiological saline retention capacity X | Water absorption capacity under a load of 4.14 kPa Y | Value of non-pressurization DW after 5 minutes Z | | |
| | [%] | [mol/mol] | [g/g] | [ml/g] | [ml/g] | | |
| Example 1 | 44 | 0.078 | 45 | 21 | 54 | 66 | 99 |
| Example 2 | 44 | 0.078 | 49 | 18 | 56 | 67 | 105 |
| Example 3 | 44 | 0.078 | 57 | 16 | 50 | 73 | 107 |
| Example 4 | 44 | 0.062 | 44 | 19 | 60 | 63 | 104 |
| Example 5 | 44 | 0.062 | 49 | 16 | 51 | 65 | 100 |
| Comparative Example 1 | 38 | 0.078 | 43 | 22 | 32 | 65 | 75 |
| Comparative Example 2 | 38 | 0.078 | 49 | 18 | 36 | 67 | 85 |
| Comparative Example 3 | 44 | 0.031 | 43 | 29 | 40 | 72 | 83 |
| Comparative Example 4 | 44 | 0.031 | 49 | 26 | 40 | 75 | 89 |
| Comparative Example 5 | 44 | 0.156 | 40 | 19 | 61 | 59 | 101 |
| Comparative Example 6 | 44 | - | 48 | 10 | 56 | 58 | 104 |

[Table 2]

| Table 2 | Water-absorbent resin used | Slope absorption test of absorbent article | |
|---|---|---|---|
| | | Amount of absorption | Absorption rate |
| | | [g] | [%] |
| Example 6 | Example 2 | 464 | 97 |
| Example 7 | Example 3 | 474 | 99 |
| Example 8 | Example 4 | 464 | 97 |
| Example 9 | Example 5 | 461 | 96 |
| Comparative Example 7 | Comparative Example 2 | 440 | 92 |
| Comparative Example 8 | Comparative Example 6 | 447 | 93 |

As can be seen from the results of Tables 1 and 2, according to the present invention, it is possible to provide a water-absorbent resin having increased water retention capacity, water absorption capacity under load, and non-pressurization DW and a method for the production thereof. When the water-absorbent resin of the present invention is used, it can be expected to provide an absorber and an absorbent article having similar performances even if the amount of the water-absorbent resin used is reduced, and there is a possibility that the manufacturing cost can be reduced.

DESCRIPTION OF REFERENCE SIGNS

1: Burette part
3: Clamp
5: Conduit pipe
10a: Water-absorbent resin
11: Stand
13: Measuring stand
13a: Through-hole
15: Nylon mesh sheet
21: Burette tube
22: Cock
23: Rubber stopper
24: Cock
25: Air introducing pipe
40: Support plate
41: Stand
42: Dropping funnel
43: Balance
44: Metal tray
50: Saline solution
51: Test solution
61: Burette part
61a: Burette
61b: Rubber stopper
61c: Cock
61d: Air introducing pipe
61e: Cock
62: Conduit pipe
63: Measuring stand
64: Measuring part
64a: Cylinder
64b: Nylon mesh
64c: Weight

100: Absorbent article
$S_0$: Horizontal plane
$S_1$: Inclined plane

**Claims**

1. A method for producing a water-absorbent resin by performing reversed phase suspension polymerization of a water-soluble ethylenically unsaturated monomer in a hydrocarbon dispersion medium, the method comprising:

   a polymerization step of mixing an aqueous solution containing the water-soluble ethylenically unsaturated monomer, the hydrocarbon dispersion medium, a polymerization initiator, a dispersion stabilizer, and an internal crosslinking agent; and
   a post-crosslinking step of post-crosslinking the polymer obtained in the polymerization step,
   wherein
   the polymerization initiator comprises an azo compound and a peroxide,
   the water-soluble ethylenically unsaturated monomer has a content of acrylic acid and a salt thereof of 70 mol% or more and 100 mol% or less,
   a concentration of the water-soluble ethylenically unsaturated monomer in an aqueous solution containing the water-soluble ethylenically unsaturated monomer is 40% by mass or more, and
   a molar ratio of the internal crosslinking agent to the azo compound (the internal crosslinking agent/the azo compound) is 0.05 or more and 0.10 or less.

2. The method for producing a water-absorbent resin according to claim 1, wherein a molar ratio of the peroxide to the azo compound (the peroxide/the azo compound) is 0.1 or more and 1.0 or less.

3. The method for producing a water-absorbent resin according to claim 1 or 2, wherein in the post-crosslinking step, a molar ratio of the post-crosslinking agent to the water-soluble ethylenically unsaturated monomer (the post-crosslinking agent/the water-soluble ethylenically unsaturated monomer) is 0.00001 or more and 0.001 or less.

4. The method for producing a water-absorbent resin according to claim 1 or 2, wherein the water-absorbent resin has the following properties (A) to (C):

   (A) a total value of a physiological saline retention capacity and a water absorption capacity under a load of 4.14 kPa is 60 or more;
   (B) a total value of the physiological saline retention capacity and a value of non-pressurization DW after 5 minutes is 90 or more;
   (C) the physiological saline retention capacity is 44 g/g or more and 70 g/g or less.

5. A water-absorbent resin which is a crosslinked product of a polymer of a water-soluble ethylenically unsaturated monomer, wherein

   the water-soluble ethylenically unsaturated monomer has a content of acrylic acid and a salt thereof of 70 mol% or more and 100 mol% or less, and
   the water-absorbent resin has the following properties (A) and (B):

   (A) a total value of a physiological saline retention capacity and a water absorption capacity under a load of 4.14 kPa is 60 or more;
   (B) a total value of the physiological saline retention capacity and a value of non-pressurization DW after 5 minutes is 90 or more.

6. The water-absorbent resin according to claim 5, wherein the physiological saline retention capacity is 44 g/g or more and 70 g/g or less.

7. An absorber comprising the water-absorbent resin according to claim 5 or 6.

8. An absorbent article comprising the absorber according to claim 7.

FIG. 1

FIG. 2

FIG. 3

## INTERNATIONAL SEARCH REPORT

| International application No. |
| --- |
| **PCT/JP2022/021925** |

**A. CLASSIFICATION OF SUBJECT MATTER**

*C08F 20/06*(2006.01)i; *C08F 2/18*(2006.01)i; *A61F 13/15*(2006.01)i; *A61F 13/53*(2006.01)i; *B01J 20/26*(2006.01)i
FI: C08F2/18; C08F20/06; A61F13/15 320; A61F13/53 300; B01J20/26 D

According to International Patent Classification (IPC) or to both national classification and IPC

**B. FIELDS SEARCHED**

Minimum documentation searched (classification system followed by classification symbols)

C08F; A61F13; B01J20/26

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2020-121093 A (SUMITOMO SEIKA CHEMICALS CO., LTD.) 13 August 2020 (2020-08-13) claims, paragraphs [0022], [0023], [0025], [0028], [0037], [0038], [0042], [0043], [0051], [0052], production example 4 | 1-4 |
| Y | | 1-4 |
| Y | WO 2020/122219 A1 (SUMITOMO SEIKA CHEMICALS CO., LTD.) 18 June 2020 (2020-06-18) paragraph [0065] | 1-4 |
| Y | JP 2007-99845 A (THE PROCTER & GAMBLE COMPANY) 19 April 2007 (2007-04-19) paragraph [0093] | 1-4 |
| Y | WO 2008/126793 A1 (NIPPON SHOKUBAI CO., LTD.) 23 October 2008 (2008-10-23) paragraph [0036] | 1-4 |
| A | WO 2019/074099 A1 (SUMITOMO SEIKA CHEMICALS CO., LTD.) 18 April 2019 (2019-04-18) entire text | 1-4 |

☐ Further documents are listed in the continuation of Box C. ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **09 August 2022** | **16 August 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

International application No.

**PCT/JP2022/021925**

**Box No. III      Observations where unity of invention is lacking (Continuation of item 3 of first sheet)**

This International Searching Authority found multiple inventions in this international application, as follows:

- Invention 1: Invention in claims 1-4
- Invention 2: Invention in claims 5-8

The invention in claims 5-8 does not prescribe an intent to be obtained by a preparation method in claims 1-4, and also, even in consideration of the common technical knowledge, the invention in claims 5-8 is not considered to primarily mean to be obtained by a preparation method in claim 1.

In addition, the matter common in invention 1 and invention 2 is a "water-absorbing resin which is a crosslinked product of a polymer of water-soluble ethylenically unsaturated monomers, wherein the water-soluble ethylenically unsaturated monomers have a content of acrylic acid and its salt of 70-100 mol%," but this matter is not novel as disclosed in documents 1-5.

Therefore, there are no other same or corresponding special technical features between invention 1 and invention 2.

As described above, inventions 1 and 2 do not satisfy unity of invention.

1. JP9-143210A
2. JP9-12613A
3. JP5-17509A
4. JP3-227301A
5. JP7-116511A

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-4**

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/021925**

| Patent document cited in search report | | | Publication date (day/month/year) | Patent family member(s) | | | Publication date (day/month/year) |
|---|---|---|---|---|---|---|---|
| JP | 2020-121093 | A | 13 August 2020 | US | 2022/0015959 | A1 | |
| | | | | claims, paragraphs [0031]-[0037], [0046]-[0052], [0060], [0061], example 4 | | | |
| | | | | EP | 3896121 | A1 | |
| | | | | CN | 113166440 | A | |
| | | | | KR | 10-2021-0101246 | A | |
| WO | 2020/122219 | A1 | 18 June 2020 | US | 2022/0055014 | A1 | |
| | | | | paragraph [0071] | | | |
| | | | | EP | 3896118 | A1 | |
| | | | | CN | 113195599 | A | |
| | | | | KR | 10-2021-0101252 | A | |
| JP | 2007-99845 | A | 19 April 2007 | US | 2007/0202772 | A1 | |
| | | | | paragraph [0104] | | | |
| | | | | WO | 2007/041075 | A1 | |
| | | | | EP | 1928511 | A1 | |
| | | | | CN | 101272813 | A | |
| WO | 2008/126793 | A1 | 23 October 2008 | US | 2010/0120940 | A1 | |
| | | | | paragraph [0049] | | | |
| | | | | EP | 2130581 | A1 | |
| | | | | CN | 101641153 | A | |
| WO | 2019/074099 | A1 | 18 April 2019 | US | 2020/0299423 | A1 | |
| | | | | whole document | | | |
| | | | | EP | 3696199 | A1 | |
| | | | | KR | 10-2020-0062233 | A | |
| | | | | CN | 111225928 | A | |
| | | | | TW | 201922807 | A | |

Form PCT/ISA/210 (patent family annex) (January 2015)

**REFERENCES CITED IN THE DESCRIPTION**

**Patent documents cited in the description**

- JP H3227301 A